# EUROPEAN PATENT SPECIFICATION

(11) **EP 1 779 799 B1**
(45) Date of publication and mention of the grant of the patent: **17.12.2008**
(21) Application number: 05023516.7
(22) Date of filing: 27.10.2005
(51) Int. Cl.: A61B 17/88

(54) **Device for fixing a reference element**
Vorrichtung zur Befestigung eines Referenzelementes
Dispositif de fixation d'un élément de référence

(43) Date of publication of application: 02.05.2007
(73) Proprietor: BrainLAB AG, 85551 Heimstetten (DE)
(72) Inventor: Maier, Christian, Dr. rer.nat., 80802 München (DE); Maier, Georg, 81541 München (DE)
(74) Representative: Schwabe - Sandmair - Marx

(56) References cited:
- EP-A- 1 523 950
- US-A1- 2002 183 759
- US-A1- 2003 009 169
- US-A1- 2004 172 044
- US-A1- 2005 020 920

## Description

The present invention relates to a device for fixing a reference element in relation to a field of surgery, in particular a human or animal bone according to the preamble of claim 1.

Recent surgical procedures require improved accuracy in order to guarantee a minimally invasive operation for the benefit of the patient. Therefore, reference arrays are now used in order to be able to perform an operation within a patient's body, wherein the surgeon does not have direct visual access without a field of surgery report. Therefore, it is necessary to fix a reference array which can be made visible by means of particular sighting methods, such as X-rays, ultrasonic methods or the like, so that the surgeon is able to obtain information about the distances and shapes and operations in the field of surgery within the patient's body. It is clear that is very important that the probes, i.e. the reference element or the reference array used for orientation, cannot be shifted or displaced with respect to their originally intended location. A displacement of the reference array or of one or more reference elements would divert the surgeon from the actual situation in the field of surgery and might thus cause surgical errors.

In the prior art, there are many different devices to be used operating on an injured bone or for repairing of an injured bone. In this respect, reference is made for example to US 6,607,561, US 6,503,251, US 6,482,208, US 6,443,955, US 5,688,285, US 4,927,421 and US 4,759,350, said prior art references all being directed to the use of surgical fixing devices for fixing ligaments, bone parts, bones and so on. However, the prior art does not refer to the necessity to ensure the location of a reference element or reference array, in order to be able to perform a minimally invasive surgical operation by means of image-guided surgical operation techniques.

From US 2003/009 169 A1 a device according to the preamble of claim 1 is known. The device is used to fix a surgical navigation system to the spinal column. It consists of a lever arrangement, the levers of which can be pivoted with respect to each other. An engagement portion with the two levers is inserted between adjacent vertebrae in a space of an intervertebral disc. To fix the device, it is necessary to straddle the levers which are specialized to function as a fixing device for spine applications.

It is the object of the present invention to provide a device for fixing of a reference element or reference array with respect to a field or surgery, in particular a human bone.

This object is solved by a device according to claim 1. Advantageous embodiments are defined in the sub-claims.

The present invention is also directed to the object of providing a device for fixing other surgical instruments with respect to a field of surgery, wherein said instruments are to be secured at the particular location and prevented from being displaced during a surgical operation. In particular, the device according to the invention is useful for image guided surgical methods and for computer aided surgical methods. Vector Vision/Trauma 2.5 are known products of the applicant directed to such surgical methods. Marker or reference elements or arrays could be attached or fixed to the device according to the invention.

The advantages of the present invention are achieved by means of a device comprising: a first fixing portion for providing a mechanical resistance against pulling and/or pushing forces; a second fixing portion which is mechanically connected by a coupling structure to the first fixing portion, said second fixing portion being provided with a surface structure for securing the device against an angular force, wherein said second fixing portion can be pivoted with respect to the fixed, first fixing portion. By means of this device according to the invention, it is possible to guarantee the attachment of any surgical means, in order to ensure that displacement is not possible. In other words, the attached device can be fixed, in particular to a bone, such that the translational and rotational stability of the location of the device can be guaranteed.

The device according to the invention is employed by means of the following steps: providing an access to the field of surgery; introducing the device into the field of surgery, penetrating the bone with a first fixing portion; pivoting the second fixing portion towards a surface of the bone; and urging the surface structure of the second fixing portion against and into the surface of the bone.

One reference element or a number of reference elements, in particular a reference array, can be fixed to the device, in particular to order to allow an image-guided surgical operation method to be used.

In accordance with the invention, the first fixing portion can be a bolt, a screw or the like. In particular, a surgical Schanz screw can be used. The diameter of this screw should be about 5 mm in order to provide the device according to the invention with a sufficient strength if the first fixing portion is penetrated into the bone and in particular drilled into the bone. Preferably a bone thickness of at least 4 to 5 mm, in particular at least 5 mm, remains around the insertion. This helps to avoid the bone spalling. The screw should be drilled monocortically into the bone structure of interest.

In accordance with the invention, the second fixing portion has a first segment having a pivoting structure which is close to the coupling structure, such that the first and the second fixing portions are pivotably coupled to each other. This structure allows the second fixing portion to be rotated or pivoted towards the surface of a bone, such that it is possible to provide another fixing location without serious injury to the bone itself. By means of the first and second fixing portion, it is possible to clamp the bone between them. The first fixing portion cannot then be rotated, because of the fixing function of the second fixing portion.

The direction of the axis of the first fixing portion, e.g. the screw or Schanz screw, is preferably tangential to the bone surface, in order to ensure the subsequent attachment of the second fixing portion. If the second fixing portion has the preferred shape of a fork, the fork is inserted through the soft tissue entry portal into the body of the patient such that the two or more prongs are respectively located to the left and right of the first fixing portion, e.g. a Schanz screw and the Schanz screw rests in a U-shaped upper part of the fork.

In accordance with another preferred embodiment, the second fixing portion is provided with a second lever segment supporting the surface structure which to be urged against the surface of the bone. An offset segment is also preferably provided between the second lever segment and a first lever segment. This offset segment allows the fixation area for the first fixing portion and the surface structure at one end of the second fixing portion, i.e. of the second lever segment to be offset. It is clear that the device according to the present invention can be used for several different bones and bone shapes, and that the offset segment can therefore help to adapt the device according to the invention to different bone shapes.

In accordance with another preferred embodiment of the invention, the second fixing portion has a shoulder segment for pivotably supporting the second fixing portion during a fixing operation action. This means that, if the first fixing portion is advanced into the bone to a certain degree and comes into engagement with a stopping structure in the second fixing portion close to the coupling structure, the second fixing portion can be rotated about the shoulder segment such that the surface structure of the second fixing portion can be urged against the surface of the bone merely by drilling or screwing the first fixing portion, in particular a screw, into the bone.

In accordance with another preferred embodiment, a connecting element can be engaged with the first fixing portion, wherein the connecting element has a protrusion for extending through a recess or hole in the first lever segment, said protrusion being engaged with a fixing element for urging the surface structure towards the bone surface and for holding the surface structure in this position. The connecting element can preferably be a bushing and the protrusion can be a threaded shaft or pin. The fixing element can be realised as a thumb screw so that a considerable force can be applied by a surgeon without having to use a particular tool.

The pivoting and urging steps for the second fixing portion can be performed by adjusting the fixing element, e.g. a thumb screw, towards the first lever segment.

To summarise, the present invention provides an attachment device and an attachment method for attaching a device to a bone. Preferably, a surgical Schanz screw having a diameter of at least 5 mm and a fork-shaped second fixing portion are used. The first fixing portion or Schanz screw having a diameter of at least 4-5 mm is drilled monocortically into the bone structure. A bone thickness of at least 5 mm should remain around the first fixing portion, e.g. the Schanz screw. The direction of the screw axis should be tangential to the bone surface in order to ensure the subsequent attachment. The fork-shaped structure is inserted through the soft tissue entry portal into the patient's body such that the preferably two prongs of the fork-shaped structure can be located to the left and right of the screw, respectively. It is clear that a different number of prongs can also be used, instead of two prongs, for instance three or four prongs which can also have differently shaped offset segments which can be extended or contracted in length, etc. Such additional features could be added in order to be able to adapt the device according to the invention to different bone shapes in the human or animal body. The screw rests in a preferably U-shaped upper part of the fork or second fixing portion, i.e. in the first lever segment which is located beyond the shoulder segment and thus beyond the centre of rotation of the second fixing portion.

A cylindrical bushing can be used achieve a sufficient stability in attaching the device according to the invention. This bushing is placed over the Schanz screw. The threaded pin is connected to the bushing and extends radially outwards from the cylinder axis of the bushing, said cylinder axis also being the axis of the screw. The thumb screw or wing nut can be screwed towards the bushing in order to urge the fork towards the Schanz screw, such that the lower or second lever segment with the surface structure is urged against the surface of a bone. This then attaches the device according to the present invention, which provides a considerable resistance against pulling and pushing forces, such that the device according to the invention can be positioned in a translationally and rotationally stable manner.

A brief description of the drawings follows illustrating preferred embodiments of the invention:
- Figures 1A to C: show a top view (A), a first side view (B) and a second side view (C);
- Figure 2: shows a first embodiment of the device according to the invention, being fixed to a bone; and
- Figure 3A and 3B: show a perspective view of another embodiment of the device according to the present invention, being fixed to a bone.

In the drawings, identical or comparable elements and parts are designated by means of the same reference numbers, in order to avoid repeatedly referring to the same elements or parts.

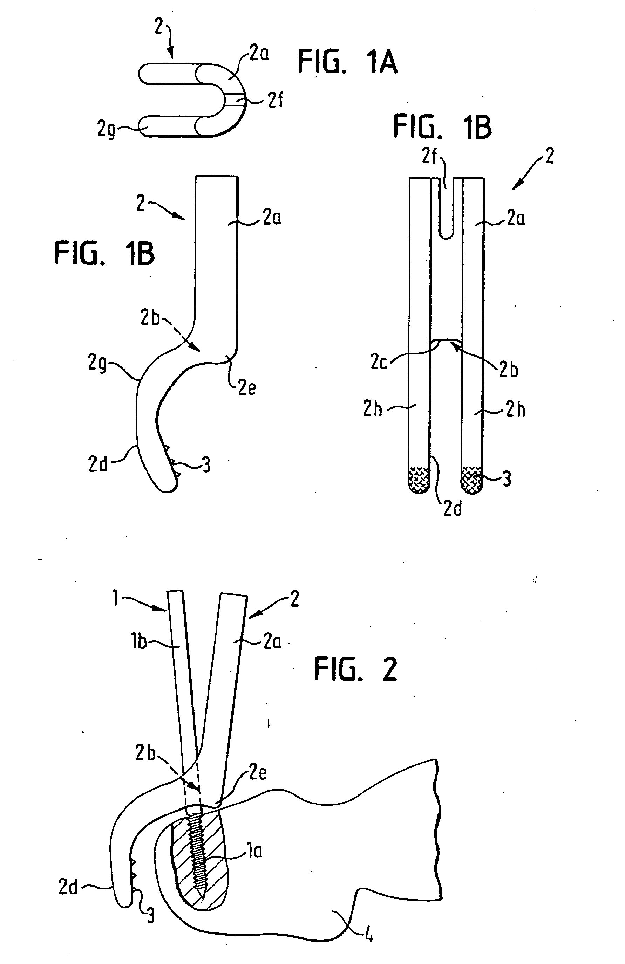

Figure 1A-1C describe a second fixing portion of a device according to the present invention. In accordance with the illustrated example of the second fixing portion 2, the portion 2 is shaped roughly like a fork, comprising a shaft 2a having a U-shaped cross section (see Figures 1A and 1C). The fork-shaped portion is provided with two prong extensions 2h which constitute the second lever segment 2d of the second fixing portion 2. The upper part of the portion 2 consists of the first lever segment 2a. The second lever segment 2d and the first lever segment 2a are connected to each other by an offset segment 2g. On the lower part of the first lever segment 2a, a shoulder segment 2e is provided which can for instance be shaped like a mandrel or a bolt. A surface structure 3 is provided at the end of the second lever segment 2d, i.e. at the end of one or both of the prong extensions 2h. This surface structure 3 can be shaped as spikes, ridges or the like, in order allow of the surface structure 3 to be securely fixed when it is attached to the surface of a bone.

The U-shaped structure of the upper or first lever segment 2a helps to secure a first fixing portion within the clearance of the U-shaped cross section of the second fixing portion 2. The centre of rotation 2b is provided close to the shoulder segment 2e, between the offset segment 2g and the first lever segment 2a.

The first lever segment 2a can also have a recess or hole 2f for attaching additional fixing elements.

In accordance with the embodiment shown in Figure 2, a first fixing portion 1 has penetrated into the bone structure, which in the present case is for example a proximal femur. The first fixing portion 1 comprises a shaft portion 1b and a threaded portion 1a which can be drilled or screwed into the tissue of the bone 4. By advancing the screw, in particular a Schanz screw, into the bone, the Schanz screw can be stopped in its axial sliding movement within the U-shaped portion of the second fork-shaped fixing portion 2 and can urge the shoulder segment 2e against the surface of the bone. Consequently, if the first fixing portion 1 is advanced further into the bone, the second lever segment 2d is rotated about the centre of rotation 2b and the shoulder segment 2e, such that the surface structure 3 is moved towards the surface of the bone and urged against the surface of the bone, in order to stably attach the device according to the present invention to the bone structure 4.

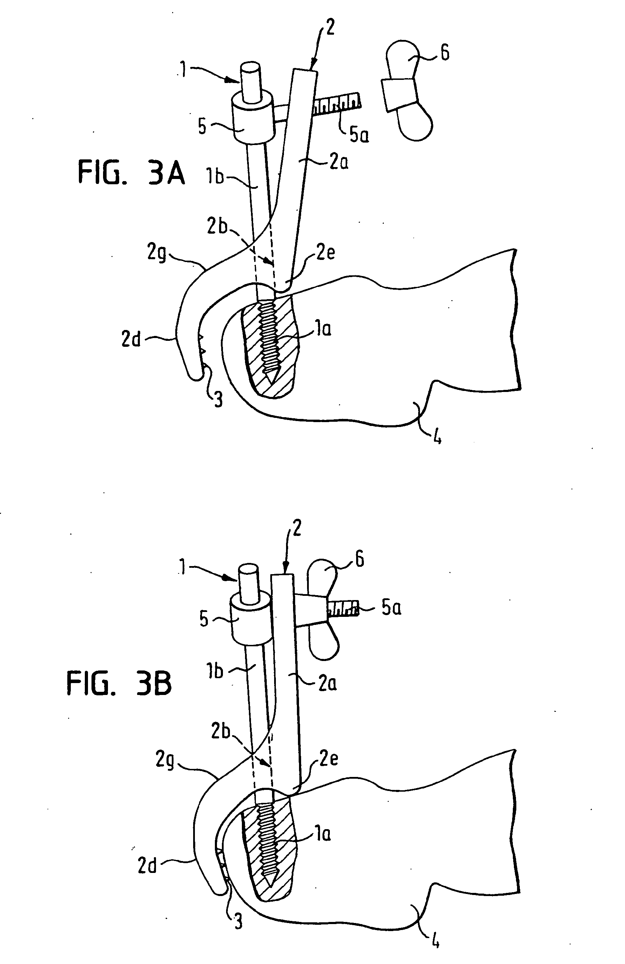

In accordance with the embodiment in Figures 3a and 3b, a connecting element 5 is additionally provided, which in this example is a bushing element 5. The cylindrical bushing element 5 is placed over the first fixing portion 1, in particular a Schanz screw, and the threaded extension 5a is extended through a recess or hole (2f in Figures 1A and 1C). A wing nut or thumb screw 6 acts as a fixing element for rotating the first lever segment 2 towards the first fixing portion 1. By means of this pivoting movement of the first lever segment 2a, the second lever segment 2d - which can consist of two or more prong extensions 2h - is rotated towards the surface of the bone structure 4. The surface structure 3 can be urged against the surface of the bone structure 4, as shown in Figure 3B.

### List of Reference signs

- 1: first fixing portion
- 1a: threaded portion
- 1b: shaft portion, lower end of the first fixing portion 1
- 2: second fixing portion
- 2a: shaft, first lever segment
- 2b: coupling structure, centre of rotation
- 2c: pivoting structure
- 2d: second lever segment
- 2e: shoulder segment
- 2f: recess or hole
- 2g: offset segment
- 2h: prong extensions
- 3: surface structure
- 4: bone
- 5: connecting element, e.g. bushing element
- 5a: protrusion, e.g. threaded extension
- 6: fixing element, e.g. wing nut or thumb screw

## Claims

1. A device for fixing a reference element with respect to a bone structure (4), comprising:
- a first fixing portion (1) for providing a mechanical resistance against pulling and/or pushing forces;
- a second fixing portion (2) which is mechanically connected by a coupling structure (2b) to the first fixing portion (1), said second fixing portion (2) being provided with a surface structure (3) in order to secure the device against an angular force;
- wherein said second fixing portion (2) can be pivoted with respect to the fixed, first fixing portion (1);
- wherein said second fixing portion (2) comprises a first lever segment (2a) with a pivoting structure (2c) close to the coupling structure (2b), such that the first and second portions (1, 2) are pivotably coupled with each other;
**characterized in that**
- said first fixing portion (1) is a bolt or a screw, in particular a surgical Schanz screw, to be introduced into the bone structure.

2. The device according to claim 1, wherein said second fixing portion (2) has a second lever segment (2d) for supporting the surface structure (3).

3. The device according to anyone of claims 1 or 2, wherein said second fixing portion (2) has a shoulder segment (2e) for pivotably supporting the second fixing portion during a fixing operation.

4. The device according to anyone of claims 1 to 3, wherein a connecting element (5) is engaged with the first fixing portion (1), said connecting element (5) having a protrusion (5a) for extending through a recess or hole (2f) in the first lever segment (2a), said protrusion (5a) being engaged with a fixing element (6) for pivotably urging the surface structure (3) towards the surface of the bone (4).

5. The device according to anyone of claims 1 to 4, wherein the first and/or second fixing portion (1, 2) is/are provided with at least one of said segments.

6. The device according to anyone of claims 1 to 5, wherein the second fixing portion (2) is formed to have at least two prong extensions (2h), extending on respective sides of the first fixing portion (1) towards the lower end (1b) of the first fixing portion (1).

## Patentansprüche

1. Vorrichtung zur Fixierung eines Referenzelements bezüglich einer Knochenstruktur (4), wobei die Vorrichtung Folgendes umfasst:
- einen ersten Fixierabschnitt (1) zur Bereitstellung eines mechanischen Widerstands gegen Zieh- und/oder Schubkräfte;
- einen zweiten Fixierabschnitt (2), der über eine Kopplungsstruktur (2b) mit dem ersten Fixierabschnitt (1) mechanisch verbunden ist und mit einer Oberflächenstruktur (3) versehen ist, um die Vorrichtung gegen eine Winkelkraft zu sichern;
- wobei der zweite Fixierabschnitt (2) relativ zu dem ortsfesten ersten Fixierabschnitt (1) schwenkbar ist, oder wobei der zweite Fixierabschnitt (2) ein erstes Hebelsegment (2a) umfasst, das nahe der Kopplungsstruktur (2b) eine Schwenkstruktur (2c) aufweist, so dass der erste und zweite Fixierabschnitt (1, 2) miteinander schwenkbar gekoppelt sind;
**dadurch gekennzeichnet, dass**
- der erste Fixierabschnitt (1) ein Bolzen oder eine Schraube, insbesondere eine chirurgische Schanzschraube, ist, der bzw. die in die Knochenstruktur einzubringen ist.

2. Vorrichtung nach Anspruch 1, wobei der zweite Fixierabschnitt (2) ein zweites Hebelsegment (2d) umfasst, um die Oberflächenstruktur (3) zu stützen.

3. Vorrichtung nach einem der Ansprüche 1 oder 2, wobei der zweite Fixierabschnitt (2) ein Schultersegment (2e) umfasst, um den zweiten Fixierabschnitt während einer Fixieroperation schwenkbar zu stützen.

4. Vorrichtung nach einem der Ansprüche 1 bis 3, wobei in den ersten Fixierabschnitt (1) ein Verbindungselement (5) eingreift, das einen Vorsprung (5a) umfasst, der sich durch eine Ausnehmung oder ein Loch (2f) im ersten Hebelsegment (2a) erstrecken soll und in ein Fixierelement (6) ergreift, um die Oberflächenstruktur (3) in Richtung auf die Knochenoberfläche (4) schwenkbar zu schieben.

5. Vorrichtung nach einem der Ansprüche 1 bis 4, wobei der erste und/oder zweite Fixierabschnitt (1,2) mit mindestens einem der genannten Segmente versehen ist/sind.

6. Vorrichtung nach einem der Ansprüche 1 bis 5, wobei der zweite Fixierabschnitt (2) mit mindestens zwei Gabelfortsätzen (2h) ausgebildet ist, die sich auf jeweiligen Seiten des ersten Fixierabschnitts (1) in Richtung auf das untere Ende (1b) des ersten Fixierabschnitts (1) erstrecken.

## Revendications

1. Dispositif pour fixer un élément de référence par rapport à une structure osseuse (4), comprenant :
- une première portion de fixation (1) pour apporter une résistance mécanique à l'encontre de forces de traction et/ou de poussée ;
- une seconde portion de fixation (2) qui est mécaniquement connectée par une structure de couplage (2b) à la première portion de fixation (1), ladite seconde portion de fixation (2) étant pourvue d'une structure de surface (3) afin de bloquer le dispositif à l'encontre d'une force angulaire ;
- dans lequel ladite seconde portion de fixation (2) peut être pivotée par rapport à la première portion de fixation fixe (1) ;
- dans lequel ladite seconde portion de fixation (2) comprend un premier segment de levier (2a) avec une structure de pivotement (2c) proche de la structure de couplage (2b), de telle façon que la première et la seconde portion (1, 2) sont couplées en pivotement l'une avec l'autre ;
**caractérisé en ce que**
ladite première portion de fixation (1) est un boulon ou une vis, en particulier une vis chirurgicale de Schanz, destinée à être introduite dans la structure osseuse.

2. Dispositif selon la revendication 1, dans lequel ladite seconde portion de fixation (2) comprend un second segment de levier (2d) pour supporter la structure de surface (3).

3. Dispositif selon l'une quelconque des revendications 1 ou 2, dans lequel ladite seconde portion de fixation (2) possède un segment d'épaulement (2e) pour supporter en pivotement la seconde portion de fixation pendant une opération de fixation.

4. Dispositif selon l'une quelconque des revendications 1 à 3, dans lequel un élément de connexion (5) est engagé avec la première portion de fixation (1), ledit élément de connexion (5) ayant une projection (5a) destinée à s'étendre à travers un évidement ou un trou (2f) dans le premier segment de levier (2a), ladite projection (5a) étant engagée avec un élément de fixation (6) pour forcer en pivotement la structure de surface (3) vers la surface de l'os (4).

5. Dispositif selon l'une quelconque des revendications 1 à 4, dans lequel la première et/ou la seconde portion de fixation (1, 2) est/sont pourvu(e)s d'un au moins desdits segments.

6. Dispositif selon l'une quelconque des revendications 1 à 5, dans lequel la seconde portion de fixation (2) est formée de manière à présenter au moins deux extensions en forme de doigts (2h), s'étendant sur des côtés respectifs de la première portion de fixation (1) vers l'extrémité inférieure (1b) de la première portion de fixation (1).
